# EUROPEAN PATENT APPLICATION

(11) **EP 4 011 316 A1**
(43) Date of publication of application: **15.06.2022**
(21) Application number: 20306546.1
(22) Date of filing: 11.12.2020
(51) Int. Cl.: A61B 50/13, B62B 5/04, B25J 5/00, A61B 34/30

(54) **SURGICAL ROBOTIC SYSTEM**

(71) Applicant: Ecential Robotics, 38610 Gieres (FR)
(72) Inventor: LEON, Adrien, 38610 GIERES (FR); ROPP, Denis, 73400 UGINE (FR)
(74) Representative: Regimbeau

(57) **Abstract**

The invention relates to a surgical robotic system comprising:
- a base (1) ;
- a robotic arm (2) comprising a proximal end attached to the base (1) and a distal end configured to be coupled to an end-effector;
- a plurality of wheels (3) coupled to the base (1) to allow moving the base on a ground (G);
- at least one stabilization assembly (4) coupled to the base (1) and movable between:
(i) an extended position wherein said stabilization assembly bears on the ground (G) so as to at least partially support the weight of the surgical robotic system; and
(ii) a retracted position wherein said stabilization assembly (4) is distant from the ground (G);
wherein said at least one stabilization assembly (4) comprises a lockable foot (41) configured to be unlocked and moved upon application of a force onto the base (1) in a direction substantially parallel to the ground so as to disengage the stabilization assembly (4) in the extended position from the ground.

## Description

### TECHNICAL FIELD

The present invention relates to the field of robotic surgery. In particular, the present invention relates to the field of systems with a movable base for a robotic arm to be used during a surgical operation.

### TECHNICAL BACKGROUND

A surgical robotic system may be used to assist a surgeon in surgical interventions.

Typical surgical operations using robotic surgery include: implantation of orthopaedic implants such as pedicular screws in the spine, implantation of various orthopaedic implants in bones, reduction and fixation of fractures during traumatological procedures, or just positioning guides or canulae at a desired position with respect to a predetermined target.

Such a surgical robotic system may include a robotic arm comprising a proximal end attached to a base and a distal end configured to be coupled to an end-effector, for example for guiding or holding a surgical tool.

The base may comprise a plurality of wheels to allow moving the base and the robotic arm on a ground, for example between a storage location and an operative location wherein the base is placed in the vicinity of an operating table so that the range of motion of the robotic arm allows reaching a targeted axis or region to carry out the surgical intervention.

The base may advantageously comprise stabilization means configured to immobilize the base relative to the ground and at least partially support the weight of the base and the robotic arm.

Such stabilization means may comprise a plurality of feet, each actuatable by an electric actuator in a vertical direction between a retracted position wherein each foot is distant from the ground, and an extended position wherein each foot bears on the ground. In the extended position, the base may be raised as compared to the retracted position, and the wheels may no longer contact the ground. The feet are arranged in such a way as to maintain a stable position of the base onto the ground, whatever the position of the robotic arm during the surgical intervention.

Document US 10,668,633 describes a surgical robotic system comprising such stabilization means.

However, in case of a power cut, the electric actuators can no longer be activated to retract the feet and allow moving the base away from the operating table. This may raise a safety issue, since under these circumstances the medical staff is supposed to move the robotic arm away from the patient as soon as possible to avoid any harmful movement of the robotic arm.

Providing an uninterruptible power supply in the base, which would allow supplying energy to the electric actuators in case of a power cut in the operating room, does not appear to be technically feasible since this would excessively increase the weight and bulkiness of the base.

### SUMMARY OF THE DISCLOSURE

The present disclosure relates to a surgical robotic system in which the feet of the stabilization means can be retracted easily by an operator in case the electric actuators are not supplied with energy.

The robotic system comprises:
- a base;
- a robotic arm comprising a proximal end attached to the base and a distal end configured to be coupled to an end-effector;
- a plurality of wheels coupled to the base to allow moving the base on a ground;
- at least one stabilization assembly coupled to the base and movable between:
   (i) an extended position wherein said stabilization assembly bears on the ground so as to at least partially support the weight of the surgical robotic system; and
   (ii) a retracted position wherein said stabilization assembly is distant from the ground;
wherein said at least one stabilization assembly comprises a lockable foot configured to be unlocked and moved upon application of a force onto the base in a direction substantially parallel to the ground so as to disengage the stabilization assembly in the extended position from the ground.

Thus, with a simple application of a force parallel to the ground by an operator, the stabilization assembly can be disengaged from the ground and the operator can then move the base thanks to its wheels.

In the present text, "vertical" designates a direction parallel to gravity, and "horizontal" designates a direction perpendicular to the vertical direction. The ground of the operating room may thus be considered to be horizontal. The terms "upper", "lower", "upwardly" or "downwardly" have to be understood with respect to the vertical direction.

In some embodiments, each stablization assembly comprises at least one electric actuator configured to move the stabilization assembly between the retracted position and the extended position and the foot is configured to be unlocked manually by a user.

In some embodiments, the stabilization assembly comprises a rod extending vertically from the base, the lockable foot being lockably mounted onto said rod.

In some embodiments, the lockable foot when unlocked is pivotably movable relative to the rod.

In some embodiments, the lockable foot when unlocked is translationally movable relative to the rod.

In some embodiments, the lockable foot is attached to the rod by a screw passing through the rod and a nut opposite to a head of the screw, the lockable foot being locked to the rod by tightening the screw head and the nut onto a respective bearing surface of the lockable foot.

In some embodiments, the lockable foot comprises a pair of parallel grooves accommodating the screw and the nut, such that application of a force onto the base in a direction substantially parallel to the ground causes the lockable foot to move along the grooves.

In some embodiments, the grooves have a substantially U shape and when the lockable foot is locked to the rod the screw is located at the bottom of the groove, such that when the lockable foot is unlocked, said lockable foot can be moved upon application of a force onto the base in two opposite directions substantially parallel to the ground so as to disengage the stabilization assembly (4) in the extended position from the ground.

In some embodiments, the lockable foot is attached to the rod by a pin passing through the rod and articulated to a lever pivotable between a locking and a releasing position, and a nut opposite to the lever, the lockable foot being locked to the rod by tightening the nut onto a bearing surface of the lockable foot, the lever being in the locking position.

In some embodiments, the lockable foot comprises a substantially flat surface configured to rest on the ground when the stabilization assembly is in the extended position.

In some embodiments, the surgical robotic system comprises at least two stabilization assemblies.

Preferably, a center of gravity of the surgical robotic system is located inside a polygon defined by the wheels and stabilization assemblies that are in contact with the ground when the stabilization assemblies are in the extended position.

In some embodiments, the surgical robotic system comprises at least three stabilization assemblies, wherein when at least three stabilization assemblies are in the extended position, the wheels are distant from the ground.

In other embodiments, when at least one stabilization assembly is in the extended position, at least one wheel remains in contact with the ground.

### BRIEF DESCRIPTION OF THE FIGURES

Further features, embodiments and advantages of the surgical robotic system will be described in the following description, based on the appended drawings wherein:
- FIGS. 1A and 1B are perspective views of the surgical robotic system, respectively in the extended and the disengagement position of the stabilization assembly;
- FIGS. 2A and 2B are side views of the bottom of the base of the surgical robotic system of FIGS. 1A and 1B, respectively;
- FIGS. 3A and 3B are perspective views of a first embodiment of the stabilization assembly, respectively in the extended position and in the disengagement position;
- FIGS. 4A and 4B are side views of the first embodiment, respectively in the extended position and in the disengagement position;
- FIGS. 5A and 5B are side views of a second embodiment of the stabilization assembly, respectively in the extended position and in the disengagement position;
- FIGS. 6A and 6B are side views of a third embodiment of the stabilization assembly, respectively in the extended position and in the disengagement position;
- FIGS. 7A and 7B are side views of a fourth embodiment of the stabilization assembly, respectively in the extended position and in the disengagement position;
- FIGS. 8A and 8B are perspective views of a fifth embodiment of the stabilization assembly, respectively in the extended position and in the disengagement position;
- FIGS. 9A to 9C are side views of the fifth embodiment, respectively in the extended position and in both disengagement positions.

Reference signs identical from one figure to another one designate elements having a similar function, whose description may not be repeated for sake of conciseness.

### DETAILED DESCRIPTION OF EMBODIMENTS

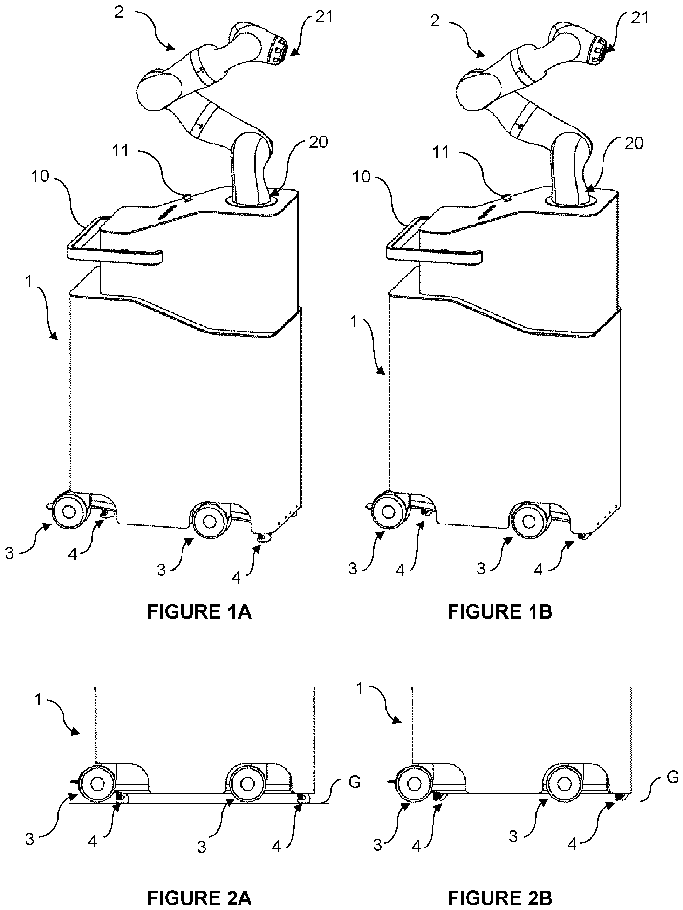

FIGS. 1A and 1B illustrate, in a non-limitative way, the general structure of the robotic system.

The robotic surgical system comprises a base 1 and a robotic arm 2.

A plurality of wheels 3 are coupled to the base 1, for example to the bottom face of the base. The wheels are arranged in such a way as to ensure stability of the base, either in a static position or in motion.

The base 1 may comprise a handle 10 that may be used by a user to manipulate the base, in particular to push or pull the base to move it on the ground. In the present text, the front side of the base is considered to be the side opposite to the handle, and the back side of the base is considered to be the handle side.

In some embodiments, the surgical robotic system may comprise four wheels, for example arranged according to a rectangle with two wheels at the front of the base and two wheels at the back of the base. Of course, a greater number of wheels and/or another arrangement of the wheels may be implemented, provided that a suitable stability of the surgical robotic system is achieved.

The robotic arm 2 comprises a proximal end 20 movably or fixedly attached to the base, a distal end 21 configured to be attached to an end-effector (not shown), and a plurality of articulated segments extending between the proximal end and the distal end.

Before and after the surgical intervention, for example when the surgical robotic system is stored or moved between the storage location and the operative location, the robotic arm may be folded so as not to protrude from the lateral faces of the base, in order not to hit obstacles when the surgical robotic system is displaced or not to form itself an obstacle for persons or objects passing in the vicinity of the surgical robotic system. Such a folded position also increases the stability of the surgical robotic system.

The surgical robotic system also comprises at least one stabilization assembly 4.

As better seen in FIGS. 2A and 2B, each stabilization assembly is movable between:
- an extended position (FIG. 2A) wherein the stabilization assembly 4 bears on the ground G so as to at least partially support the weight of the surgical robotic system; and
- a disengaged position (FIG. 2B) wherein the stabilization assembly 4 is distant from the ground G.

In the extended position, the base may be raised in the vertical direction and the wheels may no longer contact the ground. Even if the wheels remain in contact with the ground in said extended position, the stabilization assembly has the effect of immobilizing the base relative to the ground. Thus, even if a user pushes or pulls the base, for example using the handle, the base remains in a fixed position.

In the disengaged position, since the stabilization assembly is no longer in contact with the ground, a user may move the surgical robotic system, for example by pulling or pushing the base using the handle.

In preferred embodiments, each stabilization assembly comprises at least one electric actuator configured to move the stabilization assembly between the retracted position and the extended position. Each electric actuator may be connected to the mains power source. For example, the base may be electrically connected to the mains power source by an electric cable plugged into a socket in a wall of the operating room, to supply energy to all electric components arranged in the base.

For example, the base may comprise a user interface, such as a button, configured to be actuated by a user to activate the electric actuator(s) to move the stabilization assembly to the extended or the retracted position.

Preferably, when there is a plurality of stabilization assemblies, the actuators may be activated synchronously via the user interface, so that all stabilization assemblies are simultaneously moved to the extended or the retracted position.

In order to avoid having the stabilization assembly(ies) blocked in the extended position in case of a failure in powering the electric actuator(s), each stabilization assembly further comprises a foot configured to be unlocked and moved upon application of a force onto the base in a direction substantially parallel to the ground (i.e. a horizontal direction) so as to disengage the stabilization assembly in the extended position from the ground.

Preferably, said foot may be unlocked manually by a user; then, when the user applies a horizontal force onto the base, the foot is able to move so as to disengage the stabilization assembly from the ground. As a result, the base may lower smoothly to bear onto its wheels.

In some embodiments, the surgical robotic device may comprise three stabilization assemblies, for example two stabilization assemblies arranged at the front of the base and one stabilization assembly arranged at the back of the base, along the bisector of the segment joining the two front stabilization assemblies. In other embodiments, the surgical robotic device may comprise only two stabilization assemblies. In other embodiments, the surgical robotic system may comprise four stabilization assemblies or more.

In some embodiments, the wheels may leave contact with the ground when at least three stabilization assemblies are in the extended position. Otherwise, at least one wheel may remain in contact with the ground.

In general, the center of gravity of the surgical robotic system is located inside a polygon defined by the wheel(s) and stabilization assemblies that are in contact with the ground when the stabilization assemblies are in the extended position (the wheel(s) and stabilization assemblies contacting the ground forming the vertices of said polygon).

Preferably, all stabilization assemblies have the same orientation, so that application of the force onto the base once the lockable foot of each stabilization assembly has been unlocked, the application of the force in a single horizontal direction allows simultaneously disengaging all stabilization assemblies from the ground.

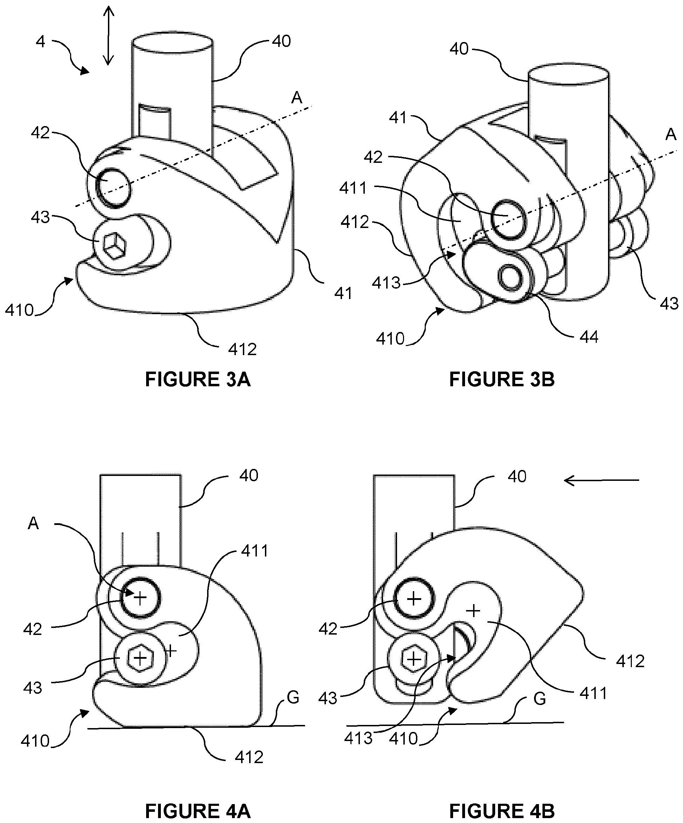

FIGS. 3A-4B illustrate a first embodiment of the stabilization assembly.

The stabilization assembly 4 comprises a rod 40 coupled to an electric actuator (not shown) so that the actuator may move the rod in a vertical direction. Said vertical direction is represented by the double arrow in FIG. 3A. The electric actuator may be embedded in the base and the rod 40 may thus extend in the vertical direction from the bottom of the base.

The stabilization assembly 4 further comprises a foot 41 movably coupled to the rod 40.

Preferably, the foot 41 is mounted at the end of the rod 40 facing the ground (the opposite end of the rod facing the bottom of the base or being located within the base). In this way, when the stabilization assembly is in the extended position, the movable foot 41 contacts the ground and supports at least partially the weight of the surgical robotic device.

In some embodiments, the foot 41 may be pivotably coupled to the rod 40 by a horizontal pin 42 and may thus be moved about a horizontal axis A between a normal position illustrated in FIGS. 3A and 4A, and a disengagement position illustrated in FIGS. 3B and 4B.

The foot 41 may comprise a substantially flat surface 412 intended to bear onto the ground when the stabilization assembly is in the extended position.

In normal operation of the surgical robotic system (i.e. when each actuator is powered), whether the stabilization assembly is in the retracted or the extended position, the movable foot 41 is locked to the rod 40 in the position illustrated in FIGS. 3A and 4A.

In some embodiments, the stabilization assembly may comprise a tightening screw 43 parallel to the pin 42 and passing through the rod 40. The screw 43 has a head configured to abut a first bearing surface 411 of the foot. As better seen in FIG. 4B, the screw is accommodated in a first groove 413 formed inside the first bearing surface 411.

The tightening screw 43 is coupled to a nut 44 attached at the end of the screw 43 opposite to the head. The nut 44 may be accommodated in a second groove 413 of the foot 41 (visible in FIG. 3B) having a matching shape and be configured to abut a second bearing surface 411 of the foot, said second bearing surface being opposite to the first bearing surface 411 with respect to the rod 40. In some embodiments, the nut 44 may have a bean shape and the second groove 413 may have a corresponding curved shape extending between an open end and a closed end. The first and second grooves 413 are parallel to each other.

In normal operation (FIGS. 3A and 4A), the head of the screw 43 frictionally engages the first bearing surface 411 and the nut 44 frictionally engages the second bearing surface 411, thereby preventing any pivoting of the foot 41 relative to the rod 40. The nut 44 is in the vicinity of the closed end of the second groove 413 of the foot 41.

Unscrewing the screw 43 from the nut 44 allows releasing the frictional force exerted by the head of the screw 43 onto the first bearing surface 411 and the frictional force exerted by the nut 44 onto the second bearing surface 411, i.e. unlocking the foot 41 from the rod 40. As a result, the foot is pivotably movable relative to the rod 40, according to a movement guided by the sliding of the nut 44 and the screw 43 inside the respective grooves 413 toward the open end.

Such unscrewing may be carried out by a user in case the actuators of the stabilization assemblies are no longer powered, and it is desired to move the surgical robotic system away from the operating table. In some embodiments, the user may use a screwdriver fitting a corresponding recess in the head of the screw. In other embodiments (not shown), the screw head may comprise a grip area allowing a user to manipulate the screw without requiring any tool. In other embodiments (not shown), the screw may include a lever pivotable between a locking position impeding any movement of the screw and an unlocking position allowing a user to unscrew the screw by rotating the lever.

The movement of the unlocked foot 41 may be triggered by a user exerting a force in a horizontal direction substantially perpendicular to the axis A, from the closed end of the groove 413 to the open end of said groove. Upon application of said force, which is indicated by the horizontal arrow in FIG. 4B, the substantially flat surface 412 of the foot 41 disengages from the ground and the foot 41 pivots about the axis A. At the end of the pivoting motion (see FIGS. 3B and 4B), the foot 41 is no longer in contact with the ground G. Thanks to the curved surface 410 provided adjacent the surface 412, the pivoting of the foot 41 is smooth and does not cause the base to suddenly fall down onto its wheels.

In the position shown in FIG. 4B, the stabilization assembly no longer supports the weight of the surgical robotic system, which rests on the ground onto its wheels. A user may thus move the surgical robotic system on the wheels.

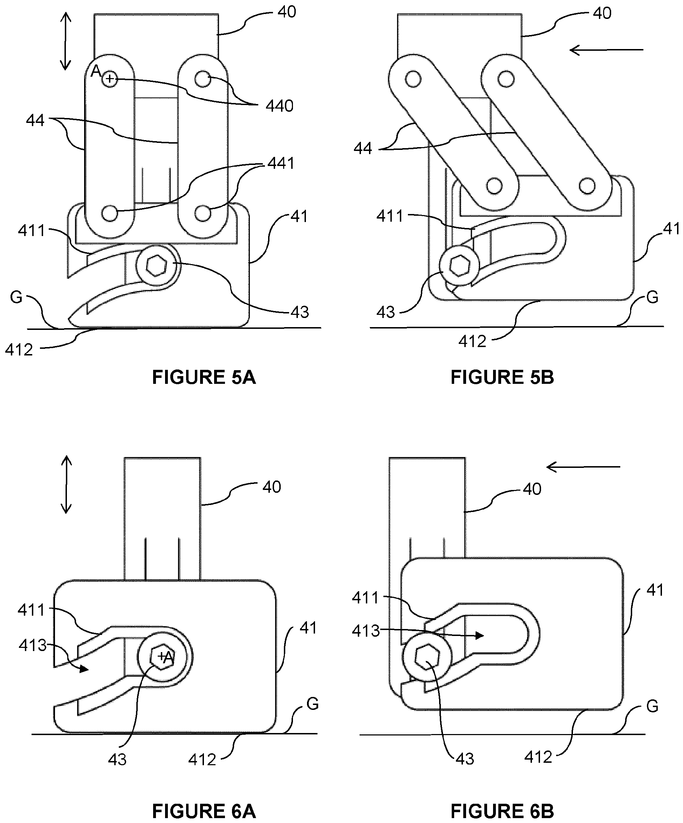

FIGS. 5A and 5B illustrate a second embodiment of a stabilization assembly, respectively in the normal extended position and in the disengagement position.

The stabilization assembly 4 comprises a rod 40 coupled to an electric actuator (not shown) so that the actuator may move the rod in a vertical direction. Said vertical direction is represented by the double arrow in FIG. 5A. The electric actuator may be embedded in the base and the rod 40 may thus extend in the vertical direction from the bottom of the base.

The stabilization assembly 4 further comprises a foot 41 movably coupled to the rod 40.

Preferably, the foot 41 is mounted at the end of the rod 40 facing the ground (the opposite end of the rod facing the bottom of the base or being located within the base). In this way, when the stabilization assembly is in the extended position, the movable foot 41 contacts the ground and supports at least partially the weight of the surgical robotic device.

In some embodiments, the foot 41 may be coupled to the rod 40 by two pairs of parallel arms 44, each arm being pivotably mounted on the foot 41 and the rod 40 by horizontal pins. Only one pair of parallel arms is visible in FIGS. 5A-5B, the other pair being on the opposite side of the rod 40. As a result, the foot 41 is movable relative to the rod 40 according to a translation in an oblique direction relative to the vertical direction.

The foot 41 may comprise a substantially flat surface 412 intended to bear onto the ground when the stabilization assembly is in the extended position.

In normal operation of the surgical robotic system (i.e. when each actuator is powered), whether the stabilization assembly is in the retracted or the extended position, the movable foot 41 is locked to the rod 40 in the position illustrated in FIG. 5A.

In some embodiments, as in the first embodiment, the stabilization assembly may comprise a tightening screw 43 parallel to the pins and passing through the rod 40. The screw 43 has a head configured to abut a first bearing surface 411 of the foot. The tightening screw 43 is coupled to a nut (not shown) attached at the end of the screw 43 opposite to the head. As previously explained with respect to FIG. 3B, the nut may be accommodated in a groove of the foot 41 having a matching shape. In some embodiments, the nut may have a bean shape and the groove may have a corresponding curved shape extending between an open end and a closed end, the open end being lower than the closed end.

In normal operation (FIG. 5A), the head of the screw 43 frictionally engages the first bearing surface 411 and the nut frictionally engages a second bearing surface opposite to the first bearing surface with respect to the rod 40, thereby preventing any movement of the foot 41 relative to the rod 40.

Unscrewing the screw 43 from the nut allows releasing the frictional force exerted by the head of the screw 43 onto the first bearing surface 411 and the frictional force exerted by the nut onto the second bearing surface, i.e. unlocking the foot 41 from the rod 40. As a result, the foot 41 is translationally movable relative to the rod 40, according to a movement guided by the sliding of the nut and the screw inside respective grooves toward the open end.

Such unscrewing may be carried out by a user in case the actuators of the stabilization assemblies are no longer powered, and it is desired to move the surgical robotic system away from the operating table. In some embodiments, the user may use a screwdriver fitting a corresponding recess in the head of the screw.

In other embodiments (not shown), the screw head may comprise a grip area allowing a user to manipulate the screw without requiring any tool. For example, the screw may be a knurled screw, with a head presenting indentations, such as axial straight lines, or lines forming a grid pattern or any other pattern.

According to an embodiment, the stabilization assembly may comprise a locking pin with a handle comprising a smooth axle passing through the rod 40, with locking balls located at the distal end of said smooth axle, engaging corresponding recesses on the other side of the rod 40 and through the foot 41, opposite to the handle, so as to prevent the rotation of the foot 41 relative to the rod 40. The handle located at the proximal end of said smooth axle may comprise a button which, when pressed, releases said locking balls for pulling said locking pin and unlocking the foot 41 from the rod 40. In such case, the above-described nut is not necessary.

According to another embodiment, the stabilization assembly may comprise a quick clamping system, comprising a pin with a lever at its proximal end to loosen the locking mechanism. The distal end of the pin is threaded and coupled to the above-described nut. The lever is pivotable between a locking position in which the lever is substantially perpendicular to the pin and prevents rotation of the pin, thereby allowing tightening the nut onto the corresponding bearing surface of the foot, and a releasing position in which the lever extends substantially along the axis of the pin and allows rotating the pin to unscrew the distal end from the nut. This clamping system has the advantage of not needing tools to loosen or tighten the locking mechanism.

The movement of the unlocked foot 41 may be triggered by a user exerting a force in a horizontal direction substantially perpendicular to the axis A, from the closed end of the groove to the open end of said groove. Upon application of said force, which is indicated by the horizontal arrow in FIG. 5B, the substantially flat surface 412 of the foot 41 disengages from the ground and is raised in the vertical direction while remaining parallel to the ground G. At the end of the pivoting motion (see FIG. 5B), the foot 41 is no longer in contact with the ground G. In this position, the stabilization assembly no longer supports the weight of the surgical robotic system, which rests on the ground onto its wheels. A user may thus move the surgical robotic system on the wheels.

FIGS. 6A and 6B illustrate a third embodiment of a stabilization assembly, respectively in the normal extended position and in the disengagement position.

The stabilization assembly 4 comprises a rod 40 coupled to an electric actuator (not shown) so that the actuator may move the rod in a vertical direction. Said vertical direction is represented by the double arrow in FIG. 6A. The electric actuator may be embedded in the base and the rod 40 may thus extend in the vertical direction from the bottom of the base.

The stabilization assembly 4 further comprises a foot 41 movably coupled to the rod 40.

Preferably, the foot 41 is mounted at the end of the rod 40 facing the ground (the opposite end of the rod facing the bottom of the base or being located within the base). In this way, when the stabilization assembly is in the extended position, the movable foot 41 contacts the ground and supports at least partially the weight of the surgical robotic device.

In some embodiments, the foot 41 may be coupled to the rod 40 by a screw 43 extending along a horizontal axis A and a nut (not visible) guided in corresponding first and second grooves 413 of the foot 41, each groove comprising a horizontal portion and an oblique portion extending downwardly from the horizontal portion. As a result, the foot 41 is movable relative to the rod 40 according to a translation in an oblique direction relative to the vertical direction.

The foot 41 may comprise a substantially flat surface 412 intended to bear onto the ground when the stabilization assembly is in the extended position.

In normal operation of the surgical robotic system (i.e. when each actuator is powered), whether the stabilization assembly is in the retracted or the extended position, the movable foot 41 is locked to the rod 40 in the position illustrated in FIG. 6A.

In some embodiments, as in the first embodiment, the screw 43 may be tightened so that its head abuts a first bearing surface 411 of the foot 41 extending around the first groove 413 and the nut abuts a second bearing surface of the foot opposite to the first bearing surface with respect to the rod 40.

In normal operation (FIG. 6A), the head of the screw 43 frictionally engages the first bearing surface 411 and the nut frictionally engages the second bearing surface, thereby preventing any movement of the foot 41 relative to the rod 40.

Unscrewing the screw 43 from the nut allows releasing the frictional force exerted by the head of the screw 43 onto the first bearing surface 411 and the frictional force exerted by the nut onto the second bearing surface, i.e. unlocking the foot 41 from the rod 40. As a result, the foot 41 is translationally movable relative to the rod 40, according to a movement guided by the sliding of the screw inside the groove toward the open end.

Such unscrewing may be carried out by a user in case the actuators of the stabilization assemblies are no longer powered, and it is desired to move the surgical robotic system away from the operating table. In some embodiments, the user may use a screwdriver fitting a corresponding recess in the head of the screw. In other embodiments (not shown), the screw head may comprise a grip area allowing a user to manipulate the screw without requiring any tool. In other embodiments (not shown), the screw may include a lever pivotable between a locking position impeding any movement of the screw and an unlocking position allowing a user to unscrew the screw by rotating the lever.

The movement of the unlocked foot 41 may be triggered by a user exerting a force in a horizontal direction substantially perpendicular to the axis A, from the closed end of the groove to the open end of said groove. Upon application of said force, which is indicated by the horizontal arrow in FIG. 6B, the substantially flat surface 412 of the foot 41 disengages from the ground and is raised in the vertical direction while remaining parallel to the ground G. At the end of the pivoting motion (see FIG. 6B), the foot 41 is no longer in contact with the ground G. In this position, the stabilization assembly no longer supports the weight of the surgical robotic system, which rests on the ground onto its wheels. A user may thus move the surgical robotic system on the wheels.

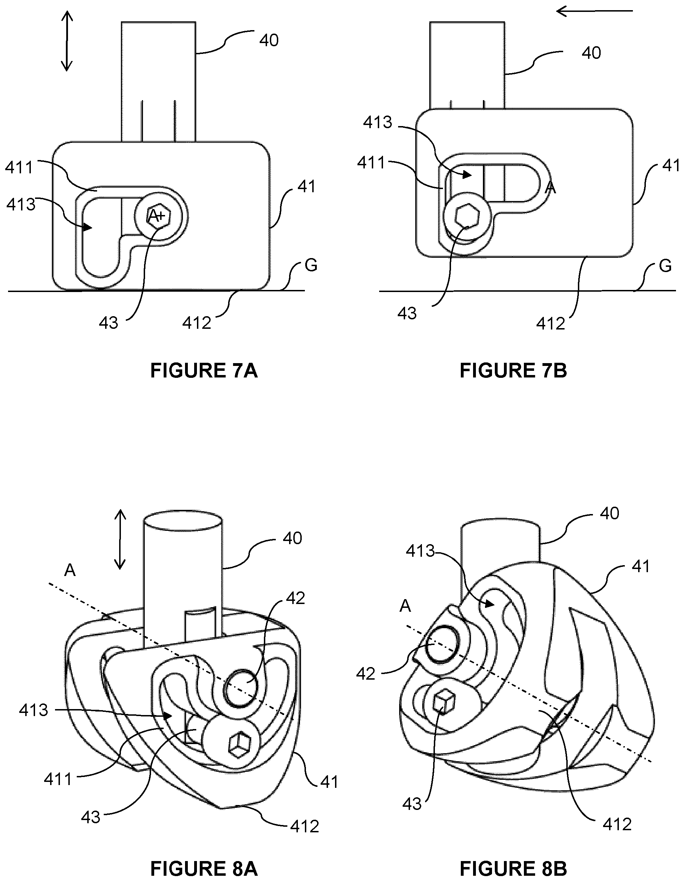

FIGS. 7A and 7B illustrate a fourth embodiment of a stabilization assembly, respectively in the normal extended position and in the disengagement position.

This fourth embodiment is substantially identical to the third embodiment, apart from the fact that the groove has a horizontal portion and a vertical portion oriented downwardly from the horizontal portion, and that both ends of the groove are closed.

In normal operation (FIG. 7A), the head of the screw 43 frictionally engages the first bearing surface 411 and the nut frictionally engages the second bearing surface, thereby preventing any movement of the foot 41 relative to the rod 40.

Unscrewing the screw 43 from the nut allows releasing the frictional force exerted by the head of the screw 43 onto the first bearing surface 411 and the frictional force exerted by the nut onto the second bearing surface, i.e. unlocking the foot 41 from the rod 40. As a result, the foot 41 is translationally movable relative to the rod 40, according to a movement guided by the sliding of the screw inside the groove toward the opposite end.

Such unscrewing may be carried out by a user in case the actuators of the stabilization assemblies are no longer powered, and it is desired to move the surgical robotic system away from the operating table. In some embodiments, the user may use a screwdriver fitting a corresponding recess in the head of the screw. In other embodiments (not shown), the screw head may comprise a grip area allowing a user to manipulate the screw without requiring any tool. In other embodiments (not shown), the screw may include a lever pivotable between a locking position impeding any movement of the screw and an unlocking position allowing a user to unscrew the screw by rotating the lever.

The movement of the unlocked foot 41 may be triggered by a user exerting a force in a horizontal direction substantially perpendicular to the axis A, from the upper end of the groove to the lower end of said groove. Upon application of said force, which is indicated by the horizontal arrow in FIG. 7B, the substantially flat surface 412 of the foot 41 disengages from the ground and is raised in the vertical direction while remaining parallel to the ground G. At the end of the pivoting motion (see FIG. 7B), the foot 41 is no longer in contact with the ground G. In this position, the stabilization assembly no longer supports the weight of the surgical robotic system, which rests on the ground onto its wheels. A user may thus move the surgical robotic system on the wheels.

In the first to fourth embodiments, the unlocked foot can be disengaged from the ground upon application of a force in a single horizontal direction.

FIGS. 8A-9C illustrate a fifth embodiment of the stabilization assembly, in which the lockable foot can be disengaged from the ground upon application of a force in two opposite horizontal directions.

The fifth embodiment is substantially similar to the first embodiment, apart from each fact that the groove has a substantially U shape with two closed ends located upper than the central bottom.

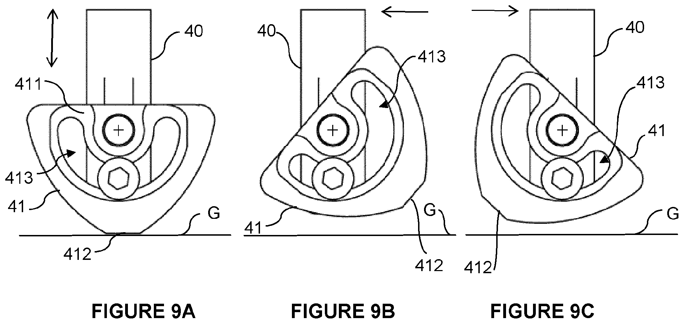

In normal operation (FIG. 9A), the head of the screw 43 is located at the bottom of the first groove 413 and frictionally engages the first bearing surface 411, and the nut is located at the bottom of the second groove and frictionally engages the second bearing surface, therey preventing any movement of the foot 41 relative to the rod 40.

Unscrewing the screw 43 from the nut allows releasing the frictional force exerted by the head of the screw 43 onto the first bearing surface 411 and the frictional force exerted by the nut onto the second bearing surface, i.e. unlocking the foot 41 from the rod 40. As a result, the foot 41 is pivotably movable relative to the rod 40, according to a movement guided by the sliding of the screw and nut within the respective groove toward either one of the closed ends of the groove.

Such unscrewing may be carried out by a user in case the actuators of the stabilization assemblies are no longer powered, and it is desired to move the surgical robotic system away from the operating table. In some embodiments, the user may use a screwdriver fitting a corresponding recess in the head of the screw. In other embodiments (not shown), the screw head may comprise a grip area allowing a user to manipulate the screw without requiring any tool. In other embodiments (not shown), the screw may include a lever pivotable between a locking position impeding any movement of the screw and an unlocking position allowing a user to unscrew the screw by rotating the lever.

The movement of the unlocked foot 41 may be triggered by a user exerting a force in either one of two horizontal directions substantially perpendicular to the axis A. Upon application of said force, which is indicated by the horizontal arrow in FIGS. 9B and 9C, the substantially flat surface 412 of the foot 41 disengages from the ground and is pivoted upwardly. At the end of the pivoting motion (see FIGS. 9B and 9C), the foot 41 is no longer in contact with the ground G. In this position, the stabilization assembly no longer supports the weight of the surgical robotic system, which rests on the ground onto its wheels. A user may thus move the surgical robotic system on the wheels.

The fifth embodiment thus provides a greater flexibility for disengaging the lockable foot from the ground, since two directions of the force to be applied onto the base are available.

Of course, the above embodiments are provided as illustrations and are not intended to be limitative. In particular, the skilled person may use other means to lock the foot to the rod, or other means to move the foot relative to the rod, without departing from the scope of the present disclosure.

## Claims

1. Surgical robotic system comprising:
- a base (1) ;
- a robotic arm (2) comprising a proximal end attached to the base (1) and a distal end configured to be coupled to an end-effector;
- a plurality of wheels (3) coupled to the base (1) to allow moving the base on a ground (G);
- at least one stabilization assembly (4) coupled to the base (1) and movable between:
(i) an extended position wherein said stabilization assembly bears on the ground (G) so as to at least partially support the weight of the surgical robotic system; and
(ii) a retracted position wherein said stabilization assembly (4) is distant from the ground (G);
wherein said at least one stabilization assembly (4) comprises a lockable foot (41) configured to be unlocked and moved upon application of a force onto the base (1) in a direction substantially parallel to the ground so as to disengage the stabilization assembly (4) in the extended position from the ground.

2. Surgical robotic system according to claim 1, wherein each stabilization assembly (4) comprises at least one electric actuator configured to move the stabilization assembly between the retracted position and the extended position and the foot (41) is configured to be unlocked manually by a user.

3. Surgical robotic system according to claim 1 or claim 2, wherein the stabilization assembly (4) comprises a rod (40) extending vertically from the base (1), the lockable foot (41) being lockably mounted onto said rod (40).

4. Surgical robotic system according to claim 3, wherein the lockable foot (41) when unlocked is pivotably movable relative to the rod (40).

5. Surgical robotic system according to claim 3, wherein the lockable foot (41) when unlocked is translationally movable relative to the rod (40).

6. Surgical robotic system according to any one of claims 3 to 5, wherein the lockable foot (41) is attached to the rod (40) by a screw (43) passing through the rod and a nut (44) opposite to a head of the screw, the lockable foot (41) being locked to the rod (40) by tightening the screw head and the nut (44) onto a respective bearing surface (411) of the lockable foot.

7. Surgical robotic system according to claim 6, wherein the lockable foot (41) comprises a pair of parallel grooves (413) accommodating the screw (43) and the nut (44), such that application of a force onto the base (1) in a direction substantially parallel to the ground causes the lockable foot (41) to move along the grooves (413).

8. Surgical robotic system according to claim 7, wherein the grooves (413) have a substantially U shape and when the lockable foot (41) is locked to the rod (40) the screw is located at the bottom of the groove, such that when the lockable foot is unlocked, said lockable foot can be moved upon application of a force onto the base (1) in two opposite directions substantially parallel to the ground so as to disengage the stabilization assembly (4) in the extended position from the ground.

9. Surgical robotic system according to any one of claims 3 to 5, wherein the lockable foot (41) is attached to the rod (40) by a pin passing through the rod and articulated to a lever pivotable between a locking and a releasing position, and a nut (44) opposite to the lever, the lockable foot (41) being locked to the rod (40) by tightening the nut onto a bearing surface (411) of the lockable foot, the lever being in the locking position.

10. Surgical robotic system according to any one of claims 1 to 9, wherein the lockable foot (41) comprises a substantially flat surface (412) configured to rest on the ground when the stabilization assembly is in the extended position.

11. Surgical robotic system according to any one of claims 1 to 10, comprising at least two stabilization assemblies (4).

12. Surgical robotic system according to claim 11, wherein a center of gravity of the surgical robotic system is located inside a polygon defined by the wheels (3) and stabilization assemblies (4) that are in contact with the ground (G) when the stabilization assemblies (4) are in the extended position.

13. Surgical robotic system according to any one of claims 1 to 12, comprising at least three stabilization assemblies, wherein when at least three stabilization assemblies (4) are in the extended position, the wheels (3) are distant from the ground.

14. Surgical robotic system according to any one of claims 1 to 13, wherein when at least one stabilization assembly (4) is in the extended position, at least one wheel (3) remains in contact with the ground.
